# EUROPÄISCHE PATENTANMELDUNG

(11) **EP 1 852 068 A2**
(43) Veröffentlichungstag der Anmeldung: **07.11.2007**
(21) Anmeldenummer: 07009014.7
(22) Anmeldetag: 04.05.2007
(51) Int. Cl.: A61B 5/145, A61B 5/0205

(54) **Verfahren und Kontrolleinrichtung für Glukosewerte**

(30) Priorität: 04.05.2006 DE 202006007261 U
(71) Anmelder: Glucotrainer GmbH & Co. KG, 86153 Augsburg (DE)
(72) Erfinder: Rundt, Markus, 86343 Königsbrunn (DE); Wolf, Arne, Dr., 86438 Kissing (DE)
(74) Vertreter: Ernicke, Klaus Stefan

(57) **Zusammenfassung**

Die Erfindung betrifft ein Verfahren und eine Einrichtung zur Kontrolle von Glukosewerten von Diabetespatienten. Die Kontrolleinrichtung weist mindestens ein Messgerät (2) für die Glukosewerte und eine Einrichtung (5) zur Auswertung und Speicherung sowie Protokollierung der Glukosewerte auf. Die Glukosewerte werden über einen Zeitraum verteilt mehrmals gemessen und in einer Messreihe mit Zeitbezug aufgenommen, ausgewertet, gespeichert und protokolliert sowie mit einem Behandlungsplan abgeglichen.

## Beschreibung

Die Erfindung betrifft ein Verfahren und eine Kontrolleinrichtung für Glukosewerte mit den Merkmalen im Oberbegriff des Verfahrens- und Vorrichtungshauptanspruchs.

In der Praxis ist es bekannt, Glukosewerte oder Blutzuckerwerte anhand von streifenartigen Proben mit einem Lesegerät zu messen. Der Anwender, insbesondere ein Diabetiker kann dabei den Glukosewert an einer mehr oder weniger detaillierten Anzeige ablesen.

Für Diabetiker besteht das Problem, dass sie sich mitunter außerhalb des tolerierten Glukosespiegels bewegen. Nur im Augenblick der invasiven Messung besteht Gewissheit über den Glukosespiegel. In der Regel hat ein Diabetiker keine Informationen über den Verlauf seiner Glukosewerte und über deren weitere Entwicklung, insbesondere über einen Entwicklungstrend. Dabei besteht das Problem, dass unerfahrene Diabetiker mangels Transparenz des Glukosestoffwechsels Lernkurven im Umgang mit ihrer Diabetes erleiden. Hierbei kommt erschwerend hinzu, dass der Diabetiker ständig verschiedene Werte im Auge behalten muss, nämlich z.B. den Glukosespiegel, die Nahrungsaufnahme, etwaige körperliche Aktivitäten, den aktuellen Gesundheitszustand, Stresssituationen, den individuellen Glukosestoffwechsel und andere Randbedingungen. Mit der Verarbeitung dieser Werte ist der Diabetiker meist überfordert. Hierbei hat der Diabetiker das Risiko, dass bei unzureichender Therapie Sekundärschäden entstehen können. Die Hilfen für den Diabetiker sind allerdings eingeschränkt. Seine Einstellung erfolgt insbesondere in einer realitätsfernen Umgebung, z.B. in einer Klinik während eines zumeist stationären Aufenthalts. Aus dieser Situation heraus ist für den Diabetiker eine realitätsnahe Planung nur schwer oder nicht erreichbar. Die Insulingabe basiert demzufolge nach Typ und Menge auf der Basis von Tabellen und einem idealisierten Glukosestoffwechsel, der in Ruhe bzw. unter Laborbedingungen bestimmt wurde und mit den Realbedingungen häufig nicht übereinstimmt. Probleme gibt es auch für den betreuenden Mediziner, weil er vom Patienten keine verlässliche Rückmeldung über die realen Lebensbedingungen und die dabei herrschenden Glukosewerte sowie die sich hieraus ergebenden Anforderungen an die Insulingabe erhält.

Es ist Aufgabe der vorliegenden Erfindung, die Kontrollmöglichkeiten für Anwender, insbesondere für Diabetiker, zu verbessern.

Die Erfindung löst diese Aufgabe mit den Merkmalen im Verfahrens- und Vorrichtungshauptanspruch.

Die beanspruchte Kontrolleinrichtung und das Kontrollverfahren haben den Vorteil, dass sie dem Anwender, insbesondere einem Patienten und dem Mediziner eine bessere und wegen des Bezugs auf die realen Lebensbedingungen verlässlichere Anzeige der Glukosewerte ermöglicht. Die Glukosewerte können längerfristig in ein oder mehreren Messreihen gesammelt, gespeichert und protokolliert werden, was dem Mediziner die realitätsbezogene Nachkontrolle und eine darauf abgestimmte bessere Medikation und Therapie erlaubt.

Andererseits können dem Anwender von der Kontrolleinrichtung Behandlungsanweisungen oder ein Behandlungsplan, insbesondere ein Ernährungs- oder Insulinplan vom Mediziner übermittelt und bei Bedarf angepasst werden.

Für den Patienten ergibt sich der Vorteil, dass er durch die vereinfachte Erfassung und Bereitstellung seiner Glukosewerte seinen individuellen Glukosestoffwechsel und dessen Veränderungen über den Tagesablauf leichter, genauer und verlässlicher lernen und sich danach in seinem Lebensablauf richten kann. Durch die leichtere Erlernbarkeit kann andererseits die Häufigkeit der Glukosemessung reduziert werden, was für den Patienten einen Komfortgewinn bedeutet. Auf der anderen Seite kann der Anwender einen an seine reale Lebenssituation besser angepassten und zeitnah aktualisierten Behandlungsplan oder auch konkrete Behandlungsanweisungen erhalten.

Aus der Erfassung und Projektion des Glukosespiegels lässt sich eine Vorhersage in die nähere Zukunft, insbesondere eine Trendinformation über drohende Hypo- und Hyperglycämien gewinnen. Hierüber lässt sich die medikamentöse Therapie, insbesondere die Insulingabe einerseits und ggf. die Nahrungsaufnahme des Patienten andererseits genauer und zielgerichteter steuern. Eine Projektion kann andererseits den Patienten rechtzeitig vor problematischen Situationen, insbesondere einer Unterzuckerung und eines Schockzustandes, warnen. Aus errechnenden drohenden Abweichungen lässt sich dies besonders gut und zielsicher vorhersagen.

Die beanspruchte Kontrolleinrichtung erfasst zumindest die Glukosewerte und erlaubt deren Auswertung und Speicherung sowie vorzugsweise auch deren Protokollierung. Dies kann zeitbezogen geschehen, wobei Uhrzeit und Datum mit dem gemessenen Glukosewert gekoppelt, gespeichert und protokolliert sowie in geeigneter Weise ausgegeben werden können.

Die Kontrolleinrichtung kann darüber hinaus die Erfassung weiterer interessanter oder bedeutsamer Werte des Patienten, insbesondere physiologischer Werte, wie Puls, Temperatur, elektrischer Hautwiderstand und dergl. ermöglichen. Diese zusätzlich erfassten Werte können mit den zeitgleich oder in einer bestimmbaren Relation erfassten Glukosewerten ebenfalls gekoppelt, gespeichert und protokolliert werden. Die physiologischen Werte sind repräsentativ für die Lebensbedingungen des Anwenders, z.B. Stresssituationen, körperliche Belastungen etc., die sich wiederum auf den Glukosespiegel und auf die Gesundheitssituation auswirken.

Der behandelnde Mediziner, der z.B. ein Arzt, insbesondere ein Diabetologe sein kann, kann aus dieser Wertematrix den Gesundheitszustand des Patienten und die Zustandsänderungen besser und genauer diagnostizieren. Insbesondere erhält der behandelnde Mediziner eine wesentlich genauer als bisher an den tatsächlichen Lebensbedingungen des Anwenders orientierte Information und kann diesen besser, effektiver und zielgerichteter mit einem direkt an die Kontrolleinrichtung zurück übermittelten und dort angezeigten Behandlungsplan oder mit konkreten, zeitbezogenen Behandlungsanweissungen therapieren. Ferner lassen sich sehr präzise Vorhersagen treffen, wobei Patient und Arzt auf etwaige Zustandsänderungen zielgerichtet und rechtzeitig reagieren können.

Der Diabetiker kann Teil eines geschlossenen Regelkreises zur Therapie seiner Krankheit auch außerhalb ständiger ärztlicher Kontrolle werden. Insbesondere kann der Diabetiker sich selbst genauer und zuverlässiger einschätzen und entsprechend zielgerichtet selbst therapieren. Die dauerhafte Protokollierung, Kontrolle, Auswertung und Projektion des Glukosespiegels verhindert schädigende Folgen für den Organismus des Diabetikers und reduziert somit Behandlungskosten. Bei Abweichungen von Normwerten wird der Diabetiker rechtzeitig gewarnt und kann reagieren. Das Diabetesmanagement wird durch eine lückenlose Datenerfassung unterstützt. Eine Unterstützung ist auch für den Notarzt bei Erstdiagnostik in Notfällen gegeben.

Die Kontrolleinrichtung kann zur Einrichtung eines solchen Regelkreises um den Anwender, insbesondere einen Diabetiker, dienen. Sie kann ihm einen neuen Stellwert für die Behandlung mitteilen. Diese kann z.B. in einer Nahrungsaufnahme oder einer Insulingabe bestehen. Durch Erstellung und Speicherung eines langfristigen Protokolls zu den Einflussgrößen, wie Nahrungsaufnahme, Insulingabe, Glukoseabbau (z.B. durch körperliche oder seelische Belastung) und dem gemessenen Glukosespiegel kann der betreuende Mediziner in der vorher erwähnten Weise Schlüsse für zukünftige Ernährungs- bzw. Insulinpläne ziehen, um den beabsichtigten Glukosespiegel sicher zu stellen. Die Datenkommunikation zwischen der Kontrolleinrichtung und dem Mediziner für die Mess- und Protokolldatenübermittlung einerseits und die Rücksendung eines Behandlungsplans oder von Behandlungsanweisungen andererseits kann auf geeigneten Datenkommunikationswegen, z.B. durch drahtlose Datenfernübertragung, erfolgen. Hierdurch Kommunikationsfehler weitgehend ausgeschaltet werden.

Die Kontrolleinrichtung kann konstruktiv in unterschiedlicher Weise ausgebildet sein. Die Kontrolleinrichtung kann insbesondere ein- oder mehrteilig sein. Für eine Erfassung der physiologischen Randbedingungen des Anwenders ist es besonders günstig, wenn die diesbezüglichen Messeinrichtungen und ggf. Speichereinrichtungen in einer Armbanduhr untergebracht sind. Dies erleichtert und verbessert die Handhabung ohne störende Wirkung auf den Anwender. Außerdem kann die Aufnahme, Verarbeitung und Speicherung von Messwerten automatisiert werden. In die Armbanduhr kann eine Mess- oder Leseeinrichtung für Proben zur Bestimmung des Glukosewertes integriert sein. Alternativ kann eine Kommunikationskopplung über Leitung oder eine drahtlose Verbindung, z.B. über Funk und entsprechende Schnittstellen erfolgen. Die Kontrolleinrichtung lässt sich dadurch für den Anwender besonders einfach, leicht, bequem und störungsarm handhaben. Für den Anwender ergibt sich ein optimaler Komfort.

In den Unteransprüchen sind weitere vorteilhafte Ausgestaltungen der Erfindung angegeben.

Die Erfindung ist in den Zeichnungen beispielsweise und schematisch dargestellt. Im einzelnen zeigen:
- Figur 1 und 2:: eine Kontrolleinrichtung mit einem Messgerät für die Glukosemessung in Front- und Seitenansicht und
- Figur 3 und 4:: ein Gerät zur Erfassung von physiologischen Messwerten in Form einer Armbanduhr in Draufsicht und Unteransicht.

Die Erfindung betrifft eine Kontrolltechnik für Glukosewerte oder Blutzuckerwerte von Anwendern, bei denen es sich z.B. um Diabetes-Patienten handelt. Dies können nicht nur Patienten mit bereits festgestellter Diabetes, sondern auch von Diabetes gefährdete und momentan noch gesunde Personen sein. Darüber hinaus ist die Kontrolltechnik für jedermann geeignet. Die Kontrolltechnik umfasst eine Kontrolleinrichtung (1) und ein Kontrollverfahren.

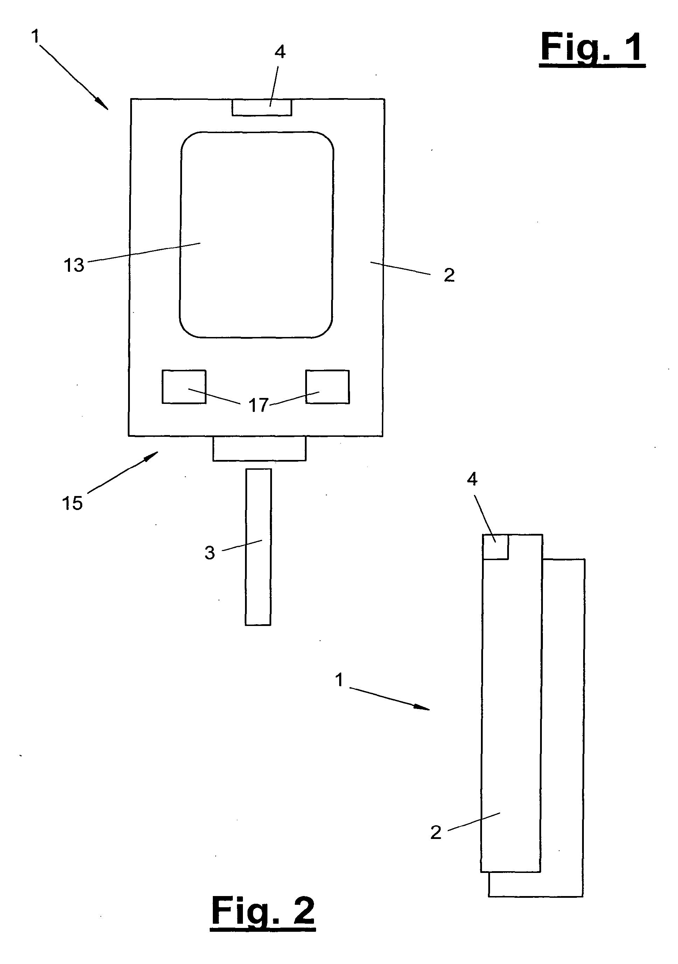

Die Kontrolleinrichtung (1) weist zumindest ein Messgerät (2) für die Glukosewerte auf, wie es beispielhaft in Figur 1 und 2 dargestellt ist. Dieses Messgerät (2) weist ein geeignetes Lesegerät für die Erfassung der Glukosewerte aus einer Probe (3), z.B. einem Probestreifen mit Urin oder dergl. des Anwenders auf. Das Messgerät (2) weist ferner eine Einrichtung (5) zur Auswertung und Speicherung der Glukosewerte auf oder ist mit dieser verbunden.

Die Kontrolleinrichtung (1) kann ferner ein oder mehrere weitere Messeinrichtungen (10,11,12) für physiologische Parameter des Anwenders oder Patienten aufweisen. Diese Parameter können z.B. die Pulsfrequenz, die Temperatur und/oder der elektrische Hautwiderstand des Anwenders sein. Die betreffende(n) Messeinrichtung(en) (10,11,12) ist oder sind mit der Auswerteeinrichtung (5) über Leitungen oder ggf. drahtlos verbunden.

Ferner kann ein Zeitgeber (14) vorhanden sein, welcher die Tageszeit und ggf. das Datum erfasst und ausgibt. Der Zeitgeber (14) ist ebenfalls mit der Auswerteeinrichtung (5) verbunden.

Die Auswerteeinrichtung (5) beinhaltet eine geeignete Recheneinheit, z.B. einen elektronischen Mikroprozessor. Sie weist ferner mindestens einen Speicher (6) für die Messdaten auf. Außerdem kann ein Programmspeicher für ein oder mehrere Programme zur Steuerung, Auswertung, Protokollierung oder sonstigen Verarbeitung der Daten vorhanden sein. Die Programme können auch Automatisierungsfunktionen beinhalten.

In der Auswerteeinrichtung (5) können die verschiedenen Messwerte miteinander gekoppelt werden. Hieraus kann eine Protokollmatrix für die gekoppelten Werte gewonnen, ggf. im Speicher (6) oder an anderer Stelle gespeichert, protokolliert und ausgegeben werden. Die Ausgabe kann in beliebig geeigneter Weise z.B. über eine Anzeige (13) oder über ein oder mehrere Schnittstellen (4,16) zur Datenkommunikation erfolgen.

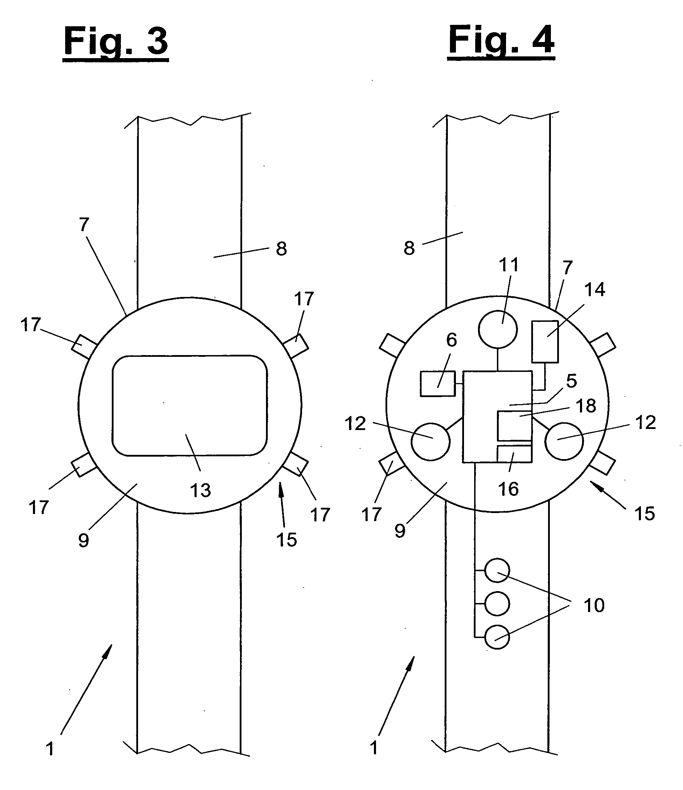

Die genannten Messeinrichtungen (10,11,12) und der Zeitgeber (14) können mit dem Messgerät (2) zu einem Gerät verbunden sein. Alternativ können getrennte Geräte (2,7) vorliegen, die ggf. aneinander über geeignete Schnittstellen angedockt werden können und miteinander kommunizieren können. Insbesondere können wie in Figur 3 und 4 dargestellt die Messeinrichtungen (10,11,12) und der Zeitgeber (14) miteinander in einem separaten Gerät (7) angeordnet sein, welches z.B. als Armbanduhr ausgebildet ist. Dieses Gerät (7) kann auch die Auswerteeinrichtung (5) nebst Speicher (6) beinhalten. Das Messgerät (2) kann als Lesegerät für Proben ausgebildet und mit einer geeigneten Steuerung sowie einer Schnittstelle (4) zur Kommunikation mit der Armbanduhr (7) ausgebildet sein.

Für die Pulsmessung ist z.B. eine Messeinrichtung (10) mit ein oder mehreren Sensoren im Armband (8) der Armbanduhr (7) untergebracht. Die Sensoren haben hierbei eine Kontaktmöglichkeit mit der Haut des Anwenders. Alternativ kann die Messeinrichtung (10) in oder an einem flexiblen Lappen mit Kontaktmöglichkeiten zur Patientenhaut untergebracht sein.

Am Gehäuse (9) der Armbanduhr (7) und insbesondere an dessen zur Patientenhaut gerichteten Innenseite können ein oder mehrere Messeinrichtungen (11,12) angebracht sein. Dies kann z.B. eine Messeinrichtung (11) mit einem Sensor zur Temperaturmessung sein. Hierdurch wird die Hauttemperatur aufgenommen. Zusätzlich kann ein Sensor zur Messung der Umgebungsbedingungen vorhanden sein. Eine andere Messeinrichtung (12) kann für die Erfassung des elektrischen Hautwiderstandes mittels zweier entsprechender Sensoren oder Elektroden an der Gehäuseinnenseite angebracht sein. Ein Zeitgeber (14) ist bei einer Armbanduhr ohnehin im Gehäuse (9) enthalten.

Wenn ausreichend Platz besteht, kann in oder an der Armbanduhr (7) und insbesondere in oder am Gehäuse (9) auch das Mess- oder Lesegerät (2) für die Proben (3) angebracht sein. Alternativ können die beiden Geräte (2,7) getrennt sein und einen Datenaustausch über ein oder mehrere geeignete Schnittstellen (4,16) durchführen. Dies können ansteckbare Kabel oder Leitungen sein. Alternativ ist eine drahtlose Verbindung, z.B. durch Funk, insbesondere Bluetooth, durch Infrarot oder auf andere geeignete Weise möglich. Alternativ ist eine lösbare Steckverbindung mittels verschließbarer elektrischer Kontakte möglich.

Die Kontrolleinrichtung (1) kann ferner eine Eingabeeinrichtung (15) aufweisen. Dies kann z.B. bei dem Messgerät (2) von Figur 1 und 2 eine Anordnung von ein oder mehreren Bedienelementen (17), insbesondere Tasten, sein. Alternativ kann die Schnittstelle (4) als Teil der Eingabeeinrichtung (15) fungieren. Ferner lassen sich über eine berührungsempfindliche Anzeige (13), z.B. ein Touch-Screen, Eingaben mittels eines Stiftes oder dergl. vornehmen. Das Messgerät (2) kann selbst eine Auswerteeinrichtung (5) der vorgenannten Art beinhalten. Es kann alternativ mit einer Steuerung ausgerüstet sein, welche die Messwertaufnahme und ggf. die Speicherung der Messwerte sowie die Datenkommunikation mit ein oder mehreren weiteren Geräten (7) ermöglicht, welche dann ein oder mehrere der genannten Auswerteeinrichtungen (5) beinhalten und die Verarbeitung und Auswertung der Messwerte ermöglichen.

Die Armbanduhr (7) kann ebenfalls an der Oberseite eine Anzeige (13) aufweisen, die unterschiedlichen Zwecken dienen kann. Zum einen können Uhrzeit und Datum angezeigt werden. Ferner können Messwerte und hieraus abgeleitete andere Werte angezeigt werden. Ferner können über die Anzeige (13) dem Anwender bzw. Patienten oder auch dem behandelnden Mediziner Informationen über Prognosen oder einen Trend des Glukosespiegels, Warnmeldungen, Therapieanweisungen oder dergl. übermittelt werden.

Die Anzeigen (13) können von beliebiger Art und Anzahl sein. Insbesondere können sie z.B. optische Anzeigen in Form von Bildschirmen, Zeilendisplays oder dergl. sein. Für die Darstellung komplexer Informationen kann eine hochauflösende und grafikfähige Pixelmatrix-Anzeige in Form eines kleinen TFT-Bildschirms oder dergl. vorhanden sein. Ansonsten kann die Anzeige (13) z.B. eine akustische Anzeige, z.B. einen Summer oder dgl. aufweisen.

Die Eingabeeinrichtung (15) kann an ein oder beiden Geräten (2,7) vorhanden sein. Bei der Armbanduhr (7) können z.B. ein oder mehrere Bedienelemente (17), z.B. in Form von Tasten, Drehstellern oder dergl. vorhanden sein. Ferner kann auch hier ein Touch-Screen Verwendung finden. Eine Dateneingabe ist auch über ein externes Gerät, z.B. eine Tastatur oder dergl. mittels der Schnittstelle (16) möglich.

Ferner kann die Kontrolleinrichtung (1) eine Datenübertragung durchführen und z.B. mit einem Telefon, insbesondere einem Handy, über die Schnittstelle(n) (4,16) kommunizieren. Die Messwerte und/oder die Auswerteergebnisse können hierüber an den Arzt oder einen anderen Empfänger übermittelt werden.

Über die verschiedenen Eingabe- und Erfassungsmöglichkeiten der Kontrolleinrichtung (1) können vom Anwender oder von anderer Stelle relevante externe Daten eingegeben werden. Z.B. kann die Insulingabe nach Typ und Menge eingegeben und der Auswerteeinrichtung (5) übermittelt werden. Diese Daten können von der Auswerteeinrichtung (5) gespeichert, protokolliert und ggf. mit den Messwerten oder den Auswerteergebnissen des Glukosespiegels und ggf. der genannten physiologischen Parameter des Anwenders verknüpft werden. Die Dateneingabe kann in vereinfachter Form über eine geeignete Menüführung mittels Vorgaben erfolgen, welche lediglich eine Bestätigung verlangen. In entsprechender Weise können Informationen über die Nahrungsaufnahme des Patienten und insbesondere die BE-Werte eingegeben werden. Die Werte der Broteinheiten (BE) kann der Patient direkt eingeben. Alternativ kann z.B. ein unerfahrener Patient die BE-Werte anhand seiner Angaben zur Nahrungsaufnahme aus externen Tabellen, z.B. aus dem Internet abfragen und direkt an die Auswerteeinrichtung (5) übermitteln lassen. Über diese Eingaben ist eine Protokollierung der Nahrungsaufnahme und der BE-Werte möglich.

Die vorerwähnten Messeinrichtungen (10,11,12) können vom Anwender mittels der Bedienelemente oder auf andere Weise mit der Eingabeeinrichtung (15) betätigt werden. Alternativ ist eine automatische Messwertaufnahme mittels eines in der Auswerteeinrichtung (5) gespeicherten Programms möglich, wobei die Messwerte auch mit Zeit und Datumsdaten gekoppelt werden können. Die Messwerte können automatisch gespeichert, protokolliert und ausgewertet werden. Ggf. können Sie automatisch angezeigt und über die Schnittstellen oder die Anzeige nach außen übermittelt werden. Durch eine programmgestützte und automatisierte Auswertung der Messdaten für den Glukosespiegel und der anderen vorerwähnten Daten, insbesondere die physiologischen Parameter und der Nahrungsaufnahme des Anwenders kann die Auswerteeinrichtung (5) den aktuellen Gesundheitszustand des Anwenders bestimmen und Prognosen für die nähere Zukunft treffen. Insbesondere kann sie den Anwender warnen, wenn er bei einem vorgegebenen Zeitplan für die Erfassung der verschiedenen Messwerte vom Plan abweicht. Solche Abweichungen können erfasst, protokolliert und per Datenfernübertragung dem behandelnden Arzt oder anderen Empfängern übermittelt werden. Ferner kann eine Warnung nebst entsprechender Informationen zur Abhilfe oder zum weiteren Verhalten des Anwenders ausgegeben werden, wenn ein kalkulierter oder gemessener Glukosewert oder sonstiger Wert außerhalb der Toleranz liegt.

Die Glukosewerte werden vom Anwender mit der Kontrolleinrichtung (1) über einen längeren Zeitraum verteilt mehrmals gemessen. Die Messwerte werden in mindestens einer Messreihe mit Zeitbezug aufgenommen, ausgewertet, gespeichert und protokolliert.

Die Auswerteeinrichtung (5) lernt ferner über die kontinuierliche Messwerterfassung über mehrere Tage den individuellen Blutzuckerstoffwechsel des Anwenders und auch die damit einhergehenden Daten und ggf. Veränderungen seiner physiologischen Randbedingungen. Aus dem gelernten Glukosestoffwechsel heraus kann von der Auswerteeinrichtung (5) eine Projektion der weiteren Entwicklung des Blutzuckerspiegels auf Grund von Nahrungsaufnahme, Insulingabe und Puls, Hauttemperatur, Hautwiderstand oder dergl. gegeben werden. Insbesondere kann automatisch auf Einflussnahmen, z.B. durch besondere Umgebungsbedingungen, Sport, Stress oder dergl. für den Anwender reagiert werden. Von der Auswerteeinrichtung (5) kann ferner eine Empfehlung für die nächste Insulingabe nach Typ und Menge berechnet und ausgegeben werden. Gleiches gilt für die Nahrungsaufnahme des Anwenders und die erforderlichen BE-Werte.

Über die Kommunikationsschnittstellen ist es ferner möglich, dass der behandelnde Arzt anhand der Messwerte und Auswertedaten einen Plan für den Anwender zur Nahrungsaufnahme, Insulingabe oder zu seinem körperlichen Verhalten, insbesondere körperlichen Belastungen, Pulsfrequenz etc. erstellt. Dies kann ein komplexer Behandlungsplan sein, der in vergefertigter Fassung niedergelegt oder als Programm gespeichert wird. Alternativ können einzelne Behandlungsanweisungen für Nahrungsaufnahme, Insulingabe oder dgl., ggf. mit Zeitbezug erstellt werden. Dies können ebenfalls feste Vorgabewerte sein. Alternativ sind dynamische Vorgabewerte möglich, die aus einem programmierten und in der Kontrolleinrichtung (1) hinterlegten Behandlungsplan unter Auswertung der aktuellen Messergebnisse von der Auswerteeinrichtung (5) selbsttätig erstellt und dem Anwender in geeigneter Weise, z.B. mit einem Warn- oder Erinnerungssignal, angezeigt werden, z.B. über die vorhandene Anzeige (13).

Dieser Plan kann an die Kontrolleinrichtung (1) per leitungsgebundener oder drahtloser Datenfernübertragung übermittelt und in die Auswerteeinrichtung (5) oder in eine separate Speichereinrichtung (18) eingegeben sowie gespeichert werden. Auf umgekehrten Wege kann der behandelnde Mediziner die Daten der Auswerteeinrichtung (5) und ihrer Speicher (6) auslesen. Über die Datenkommunikation ist ferner ein Diabetesmanagement für die Anwender oder den Arzt auf einem PC oder einer Bedienkonsole möglich.

Von der Kontrolleinrichtung (1) können im weiteren Anamnesedaten und biometrische Daten erfasst und/oder gespeichert werden. Diese Daten können einem Diabetesmanagement zugeordnet werden. Ferner kann eine Ausleseroutine für Notfälle vorhanden sein, mit denen z.B. der Notarzt Messwerte und Auswertedaten erhält, mit denen die Therapie unterstützt werden kann.

Abwandlungen der gezeigten Ausführungsformen sind in verschiedener Weise möglich. Insbesondere können die Merkmale der Ausführungsbeispiele beliebig miteinander kombiniert und ausgetauscht werden.

### BEZUGSZEICHENLISTE

- 1: Kontrolleinrichtung für Glukosewerte
- 2: Messgerät
- 3: Probe, Messstreifen
- 4: Schnittstelle, Kommunikations-Schnittstelle
- 5: Auswerteeinrichtung
- 6: Speicher
- 7: Messgerät, Uhr
- 8: Armband
- 9: Gehäuse
- 10: Messeinrichtung, Pulssensor
- 11: Messeinrichtung, Temperatursensor
- 12: Messeinrichtung, Sensor für Hautwiderstand
- 13: Anzeige
- 14: Zeitgeber
- 15: Eingabeeinrichtung
- 16: Schnittstelle, Kommunikations-Schnittstelle
- 17: Bedienelement, Taste
- 18: Speichereinrichtung für Behandlungsplan

## Patentansprüche

1. Verfahren zur Kontrolle der Glukosewerte von Anwendern, insbesondere Diabetes-Patienten, **dadurch gekennzeichnet, dass** die Glukosewerte vom Anwender mit einem Messgerät (2) einer Kontrolleinrichtung (1) über einen Zeitraum verteilt mehrmals gemessen und die Messwerte mit einer Einrichtung (5) in einer Messreihe mit Zeitbezug aufgenommen, ausgewertet, gespeichert und protokolliert werden.

2. Verfahren nach Anspruch 1, **dadurch gekennzeichnet, dass** dem Anwender von der Kontrolleinrichtung (1) Behandlungsanweisungen oder ein Behandlungsplan, insbesondere ein Ernährungs- oder Insulinplan übermittelt werden.

3. Verfahren nach Anspruch 1 oder 2, **dadurch gekennzeichnet, dass** das Protokoll der Messwerte von der Kontrolleinrichtung (1) an einen Mediziner und/oder ein Behandlungsplan vom Mediziner an die Kontrolleinrichtung (1) durch leitungsgebundene oder drahtlose Datenfernübertragung übermittelt wird.

4. Kontrolleinrichtung für Glukosewerte, **dadurch gekennzeichnet, dass** die Kontrolleinrichtung (1) mindestens ein Messgerät (2) für die Glukosewerte aufweist und mit einer Einrichtung (5) zur Auswertung und Speicherung der Glukosewerte verbunden ist.

5. Kontrolleinrichtung nach Anspruch 4, **dadurch gekennzeichnet, dass** die Kontrolleinrichtung (1) ein oder mehrere weitere Messeinrichtungen (10,11,12) für physiologische Parameter des Anwenders aufweist.

6. Kontrolleinrichtung nach Anspruch 4 oder 5, **dadurch gekennzeichnet, dass** die Messeinrichtung(en) (10,11,12) mit der Auswerteeinrichtung (5) verbunden ist/sind.

7. Kontrolleinrichtung nach Anspruch 4, 5 oder 6, **dadurch gekennzeichnet, dass** die Kontrolleinrichtung (1) eine Messeinrichtung (10) für den Puls und/oder für die Temperatur und/oder für den Hautwiderstand aufweist.

8. Kontrolleinrichtung nach einem der vorhergehenden Ansprüche, **dadurch gekennzeichnet, dass** die Kontrolleinrichtung (1) mindestens einen Zeitgeber (14) aufweist.

9. Kontrolleinrichtung nach einem der vorhergehenden Ansprüche, **dadurch gekennzeichnet, dass** die Kontrolleinrichtung (1) mindestens eine Einrichtung (18) zur Speicherung und Wiedergabe von Behandlungsanweisungen oder einem Behandlungsplan, insbesondere einem Ernährungs- oder Insulinplan aufweist.

10. Kontrolleinrichtung nach einem der vorhergehenden Ansprüche, **dadurch gekennzeichnet, dass** die Auswerteeinrichtung (5) und/oder die Speicheinrichtung (18) im oder am Messgerät (2) angeordnet ist/sind.

11. Kontrolleinrichtung nach einem der vorhergehenden Ansprüche, **dadurch gekennzeichnet, dass** die Auswerteeinrichtung (5) und/oder die Messeinrichtung(en) (10,11,12) und/oder der Zeitgeber (14) und/oder die Speicheinrichtung (18) in einem gemeinsamen tragbaren Gerät (2) angeordnet sind.

12. Kontrolleinrichtung nach einem der Ansprüche 1 bis 11, **dadurch gekennzeichnet, dass** die Auswerteeinrichtung (5) und/oder die Messeinrichtung(en) (10,11,12) und/oder der Zeitgeber (14) und/oder die Speicheinrichtung (18) in mindestens einem getrennten Gerät (7) angeordnet und mit dem Messgerät (2) über eine Kommunikations-Schnittstelle (14,16) verbunden ist/sind.

13. Kontrolleinrichtung nach einem der vorhergehenden Ansprüche, **dadurch gekennzeichnet, dass** die Geräte (2,7) über eine Leitung oder drahtlos verbunden sind.

14. Kontrolleinrichtung nach einem der vorhergehenden Ansprüche, **dadurch gekennzeichnet, dass** das Gerät (2,7) als Armbanduhr ausgebildet ist.

15. Kontrolleinrichtung nach einem der vorhergehenden Ansprüche, **dadurch gekennzeichnet, dass** die Messeinrichtung(en) (10,11,12) am Gehäuse (9) und/oder am Armband (8) der Armbanduhr angeordnet ist/sind.

16. Kontrolleinrichtung nach einem der vorhergehenden Ansprüche, **dadurch gekennzeichnet, dass** das Gerät (2,7) mindestens eine Anzeige (13) aufweist.

17. Kontrolleinrichtung nach einem der vorhergehenden Ansprüche, **dadurch gekennzeichnet, dass** das Gerät (2,7) mindestens ein Bedienelement (17) aufweist.
